(19) Europäisches Patentamt
European Patent Office
Office européen des brevets

(11) Publication number: **0 131 281 B1**

(12) **EUROPEAN PATENT SPECIFICATION**

(45) Date of publication of patent specification: **25.09.91**

(51) Int. Cl.[5]: **C07D 303/36**, C07D 301/27, C08G 59/10

(21) Application number: **84107924.7**

(22) Date of filing: **06.07.84**

The file contains technical information submitted after the application was filed and not included in this specification

(54) **Process for preparing polyglycidylamine derivatives.**

(30) Priority: **08.07.83 JP 125313/83**

(43) Date of publication of application:
**16.01.85 Bulletin 85/03**

(45) Publication of the grant of the patent:
**25.09.91 Bulletin 91/39**

(84) Designated Contracting States:
**BE DE FR GB IT NL**

(56) References cited:

**EP-A- 031 435**
**EP-A- 0 049 687**
**EP-A- 0 066 546**
**DE-A- 2 437 318**
**DE-A- 2 520 733**
**DE-A- 2 846 123**
**DE-B- 1 011 618**
**DE-C- 1 263 754**
**JP-A-23 181**
**US-A- 2 951 822**
**US-A- 3 595 882**

**J. Chem. Soc. 57, 887 (1935)**

**ORGANIKUM 15 Ed., 1976, p. 409-412**

**Chem. Abstracts, vol. 83, n 22, Dec.1, 1975, Columbus, Ohio, USA page 34, column 1, abstract-n 180 059w**

(73) Proprietor: **KANEGAFUCHI KAGAKU KOGYO KABUSHIKI KAISHA**
**2-4 Nakanoshima 3-chome**
**Kita-ku Osaka-shi Osaka-fu(JP)**

(72) Inventor: **Tomita, Haruo**
**1, Hiyodoridai 2-chome Kita-ku**
**Kobe-shi Hyogo-ken(JP)**
Inventor: **Yonezawa, Kazuya**
**9-30-406, Maikodai, 2-chome Tarumi-ku**
**Kobe-shi Hyogo-ken(JP)**

(74) Representative: **Türk, Gille, Hrabal et al**
**Brucknerstrasse 20**
**W-4000 Düsseldorf 13(DE)**

EP 0 131 281 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chem. Abstracts, vol. 98, n 1, Jan. 3, 1983, Columbus, Ohio, USA page 16, colum 22, abstract-n 5 022s

Chem. Abstracts, vol. 98, n 18, May 2, 1983, Columbus, Ohio, USA page 36, colum 1, abstract-n 144 346p

Chem. Abstracts, vol. 98, n 24, June 13, 1983, Columbus, Ohio, USA, page 504, colum 1, abstract-n 206 286e

## Description

The present invention relates to a process for preparing polyglycidylamine derivatives.

In recent years, glycidyl compounds of polyfunctional amines have come in common use as a material for pre-preg of the carbon fiber. Among those compounds, tetraglycidyl-4,4'-diaminodiphenylmethane, which is obtained by using 4,4'-diaminodiphenylmethane as a starting material prepared from aniline and formaldehyde and subjecting to glycidylization with epichlorohydrine, has an excellent property as an epoxy resin for pre-preg of the carbon fiber. However, such an epoxy resin has a drawback that the material cost is high since expensive 4,4'-diaminodiphenylmethane is used as a starting material in a preparation thereof. Prior art documents, J .Am.Chem.Soc.,57, 887 (1935) and Japanese Tokkyo Kokoku 23181/1967, are disclosing processes for the preparation of 4,4'-DDM starting from aniline and formaldehyde. From DE-C-12 63 754 a process for preparing aromatic polyisocyanates by condensation of aniline derivatives is known.

From US-A-2 591 822 and US-A-3 595 882 it is known to react aniline with an epihalohydrine and subsequently induce ring closure to yield diglycidyl compounds. The documents do not refer to the condensation reaction of the reacted aniline compounds with aldehydes.

It is an object to prepare tetraglycidyl-4-4'-diaminodiphenylmethan of high purity in a high yield with a low cost and without difficulty.

These and other objects of the present invention will become apparent from the description hereinafter.

In accordance with the present invention there is provided a process for preparing a polyglycidylamine derivative from an aniline compound, characterized by reacting an aniline compound of the formula (I):

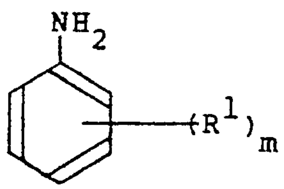

(I)

wherein $R^1$ is methyl and m is 0 or 1, provided that $R^1$ does not bond to p-position, with not less than a stoichometrical amount of an epihalohydrine at a temperature of 50° to 110° C in the presence of water in an amount of 0,1 to 10 moles per 1 mole of the aniline compound to give an addition product, subjecting the addition product to condensation reaction with an aldehyde compound or compounds of the formula (II):

$R^2$-CHO (II)

wherein $R^2$ is a hydrogen atom or an aliphatic group of $C_1$ to $C_{20}$, in the presence of HCl to give a condensation product, and treating the condensation product with alkali in the presence of a phase transfer catalyst to cause ring closure and form epoxy group.

A feature of the present invention is to carry out the condensation reaction employing the aldehyde compounds after the addition reaction employing the aniline compounds and an ephihalohydrine is completed, whereby a condensation reaction at ortho-position of the benzene ring of the aniline compounds can be inhibited and a condensation reaction at para-position of the benzene ring of the aniline compounds can be carried out with good selectivity.

Another feature of the present invention is to be able to prepare polyglycidylamine derivatives having a great variety of the structure with ease by employing the substituted anilines except aniline.

Fig. 1 is an infrared absorption spectrum of the epoxy resin prepared by the process of the present invention;

Fig. 2 is a $^1$H-NMR spectrum (80 MHz, internal standard: TMS) of the epoxy resin prepared by the process of the present invention;

Fig. 3 is an infrared absorption spectrum of the epoxy resin prepared by the process out of the scope of the invention; and

Fig. 4 is a $^1$H-NMR spectrum (80 MHz, internal standard: TMS) of the epoxy resin prepared by the process out of the scope of the invention.

First step of the process according to the present invention includes subjecting the aniline compounds and the epihalohydrine to addition reaction. The aniline compounds usable in the reaction are compounds of the formula (I):

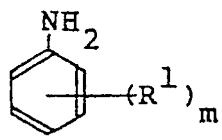

(I)

wherein $R^1$ is methyl and m is 0 or 1. Typical examples of such aniline compounds are, for instance, aniline and an alkylated aniline such as o-toluidine.

Typical examples of the epihalohydrine usable in the reaction are, for instance, epichlorohydrine, epibromohydrine and epiiodohydrine.

A used amount of the epihalohydrine is not less than a stoichiometrical amount, preferably 1.1 to 1.3 times stoichiometrical amount.

The addition reaction can be carried out without catalysts. But a catalytic amount of water is preferably added to the reaction system. In that case, the added amount of water is 0.1 to 10 moles, preferably 0.5 to 2.0 moles per 1 mole of the used aniline compounds.

The reaction can be carried out at any temperature within the range of 50 to 110°C, preferably 60 to 80°C. The reaction time varies according to the reaction temperature. The addition reaction, for example, at 80°C completes in 4 hrs.

Second step of the process according to the present invention includes subjecting the addition product obtained in the first step described above and the aldehyde compounds to condensation reaction. The aldehyde compounds usable in the reaction are compounds of the formula (II):

$R^2$-CHO (II)

wherein $R^2$ is hydrogen or an aliphatic group of $C_1$ to $C_{20}$, or its mixture. Typical examples of such aldehyde compounds are, for instance, formaldehyde; an aliphatic aldehyde such as $CH_3CHO$, $CH_3CH_2CHO$,

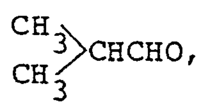

$CH_3$ $CH_2CH_2CH_2CHO$ or

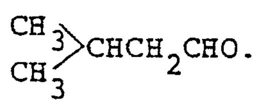

A used amount of the aldehyde compounds is not less than 0.5 mole per 1 mole of the used aniline compounds. By using 1 mole of the aniline compounds and 0.5 mole of the aldehyde compounds, a condensation product at para-position consisting of two molecules of the aniline compounds and one molecule of the aldehyde compounds is obtained quantitatively.

As a catalyst of the condensation reaction, there is added, for instance, an inorganic acid such as hydrochloric acid or phosphoric acid; or an organic acid such as formic acid, oxalic acid or acetic acid. An added amount of the exemplified acids as a catalyst is 0.5 to 1.5 moles, preferably 0.8 to 1.2 moles per 1 mole of the used aniline compounds.

The reaction generally completes in 2 to 5 hrs at 70 to 100°c.

The third step of the process according to the present invention includes treating the condensation product obtained in the second step described above with alkali, whereby the condensation product is subjected to dehydrogenhalide reaction of the halohydrine residue and the used acid is neutralized. Typical examples of the alkali usable in the present invention are, for instance, sodium hydroxide, potassium hydroxide, sodium carbonate, and potassium carbonate. The alkalis exemplified above can be used in the form of a solid or an aqueous solution of arbitrary concentrations.

A used amount of the alkali is a sum of the moles of the acid used in the second step and 1 to 1.5 times moles stoichiometrically necessary for the dehydrogenhalide reaction of the halohydrine residue. The reaction completes in 1 to 2 hrs at 50 to 60°C.

There may be employed a phase transfer catalyst to accelerate the dehydrogenhalide reaction. Typical examples of the phase transfer catalyst usable in the reaction are, for instance, a quarternary ammonium salt such as tetrabutylammonium bromide, trioctylmethylammonium chloride or benzyltriethylammonium chloride; a quarternary phosphonium salt such as tetraphenylphosphonium chloride or triphenyl methylphosphonium chloride; or a quarternary arsonium salt.

The exemplified catalysts are used in any amount within the range of 0.01 to 50 % mole, preferably 0.1 to 5 % mole of the used aniline compounds. When the phase transfer catalyst is employed, the dehydrogenhalide reaction can be completed in a shorter time and at a lower temperature, whereby polyglycidylamine compounds of higher purity are obtained.

After the completion of the third step, the obtained product is washed with water and unreacted epihalohydrine is removed by distillation to give polyglycidylamine compounds of high purity.

Especially when aniline and formaldehyde are used as starting materials, almost pure N,N,N',N'-tetraglycidyl-4,4'-diaminodiphenylmethane can be prepared easily with low cost as a product.

As described above, according to the process of the present invention, polyglycidylamine derivatives of high purity can be prepared in one-pot reaction from the cheap aniline compounds as a starting material in a high yield.

In a conventional process, at a time of condensing aniline with formaldehyde, polyamine compounds having more than three amine groups are also produced as a by-product since formaldehyde reacts at the ortho-position as well as the para-position of the benzene ring of aniline.

On the contrary, according to the process of the present invention, N,N,N'N'-tetraglycidyl-4,4'-diaminodiphenylmethane can be prepared in a yield more than 95 % based on the used aniline, and therefore it is advantageous.

The polyglycidylamine compounds prepared by the process of the present invention is substantially the same as the polyglycidylamine compounds prepared from 4,4'-diaminodiphenylmethane or the derivatives thereof as a starting material.

The polyglycidylamine derivatives prepared according to the process of the present invention can be cured by a conventional known curing agent such as polyamine, an acid anhydride or polyphenol to give a cured material having a high heat resistance.

The present invention is more specifically described and explained by means of the following Examples.

Example 1

18.6 g ( 0.2 mole) of aniline, 40.7 g (0.44 mole) of epichlorohydrine and 3.6 g (0.2 mole) of water were mixed and stirred for 5 hrs at 80°. After cooling the reaction mixture to 50°C, 23.03 g of 38 % conc. hydrochloric acid was gradually added to the mixture to give a homogeneous solution. The thus obtained solution was maintained at 70°C and 8.11 g of a 37 % aqueous solution of formaldehyde (0.1 mole of formaldehyde) was added dropwise to the solution for 10 mins with stirring. Then, the stirring was continued for another 4 hrs at 80°C.

After cooling the reaction solution to 50°C, 1.13 g (0.005 mole) of benzyltriethylammonium chloride was added to the solution. Then, 144 ml of a 5N aqueous solution of sodium hydroxide (0.72 mole of sodium hydroxide) was added to the solution for 30 mins with stirring and the stirring was continued for another 2 hrs at 50°C. The obtained reaction solution was cooled to room temperature, whereby the solution separated in an aqueous layer and an organic layer. The aqueous layer was discarded, and the organic layer was washed 3 times with 300 ml of water. Unreacted epichlorohydrine was distilled out by means of an evaporator to give 40.3 g of N,N,N',N'-tetraglycidyl-4,4'-diaminodiphenylmethane in the form of a viscous pale brown liquid (yield: 95 %).

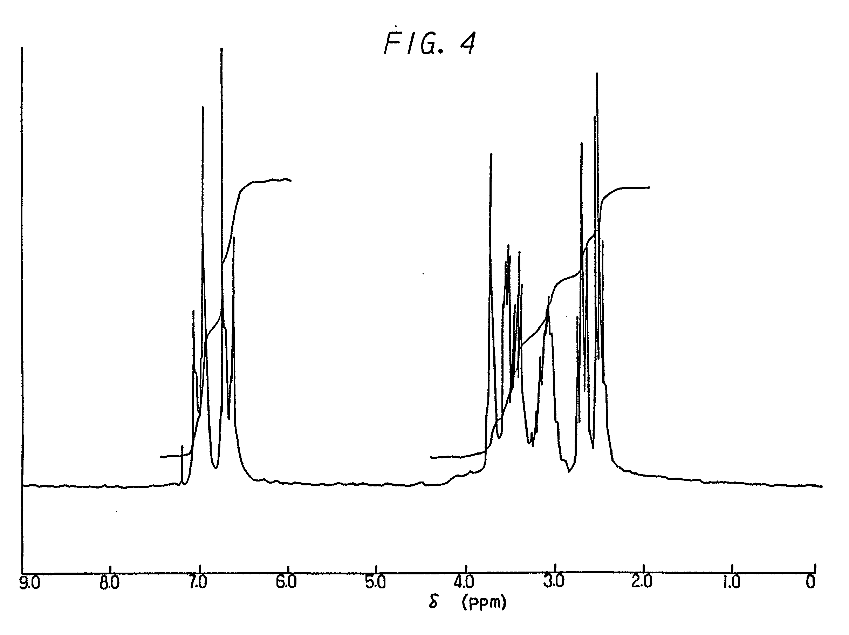

An infrared absorption spectrum and a $^1$H-NMR specturm of the thus obtained product are shown in Fig. 1 and Fig. 2, respectively, and agree with those of the product prepared in Reference Example shown in Fig. 3 and Fig. 4, respectively.

An epoxy equivalent of the product measured by HCl-pyridine method was 123 (calculated value: 106).

Example 2

18.6 g (0.2 mole) of aniline, 40.7 g (0.44 mole) of epichlorohydrine and 3.6 g (0.2 mole) of water were mixed and stirred for 5 hrs at 80°C. After cooling the reaction mixture to 60°C, 26.58 g of 38 % conc. hydrochloric acid (0.277 mole of hydrochloric acid) was gradually added to the mixture to give a homogeneous solution. The thus obtained solution was maintained at 70°C and 8.11 g of a 37 % aqueous

solution of formaldehyde (0.1 mole of formaldehyde) was added to the solution for 10 mins with stirring. Then, the stirring was continued for another 4 hrs at 90 to 95°C.

After cooling the reaction solution to 60°C, 56 ml of a 5N aqueous solution of sodium hydroxide (0.28 mole of sodium hydroxide) was added to the solution with stirring, whereby a viscous organic compound was precipitated. The aqueous layer was discarded by decantation. To the viscous product was added a mixture of 120 ml of a 5N aqueous solution of sodium hydroxide (0.6 mole of sodium hydroxide) and 2.27 g (10 mmole) of benzyltriethylammonium chloride for 20 mins at 50°C with stirring. Then, the stirring was continued for another 1 hr at 60 to 65°C. The resulting reaction mixture was cooled to room temperature, whereby the mixture separated into an aqueous layer and an organic layer. The aqueous layer was discarded and the organic layer was washed 3 times with 300 ml of water. Unreacted epichlorohydrine was distilled out at 100°C under a pressure of 3 mmHg to give 41.55 g of the polyglycidylamine compound in the form of a viscous brown liquid (yield: 98.3 %).

With respect to the thus obtained product, an epoxy equivalent was 127 and an infrared absorption specturm agreed with that of the product in Example 1.

Example 3

The addition reaction was carried out employing aniline and epichlorohydrine exactly according to the procedure described in Examples 1 and 2. To the obtained addition product was added 26.58 g of 38 % conc. hydrochloric acid (0.277 mole of hydrochloric acid) and then 5.81 g (0.1 mole) of propionaldehyde dropwise at 70 to 75°C. Then, the stirring was continued for another 4 hrs at 80 to 85°C.

After cooling the reaction mixture to 60°C, 69.3 ml of a 4N aqueous solution of sodium hydroxide (0.277 mole of sodium hydroxide) was added to the mixture. The resulting mixture was stirred for some while. After standing, an aqueous layer was discarded by decantation. To the organic layer was added 2.27 g (10 mmol) of benzyltriethylammonium chloride and then 50 g of a 40 % aqueous solution of sodium hydroxide (0.5 mole of sodium hydroxide) with stirring. The admixture was vigorously stirred for 45 mins at 60°C and cooled to room temperature. 200 ml of water was added to the admixture so that sodium chloride was precipitated. After dissolving sodium chloride, an aqueous layer was discarded. The organic layer was washed 3 times with 300 ml of water, and unreacted epichlorohydrine was distilled out at 100°C under a pressure of 3 mmHg to give 44.09 g of the polyglycidylamine compound in the form of a pale brown liquid (yield: 98 %).

An epoxy equivalent of the thus obtained product measured by HCl-pyridine method was 132. From an infrared absorption spectrum and a $^1$H-NMR spectrum, it is confirmed that the main component of the product is 3,3-bis (N,N-diglycidyl-p-aminophenyl)propane.

Reference Example

1.98 g (10 mmol) of 4,4'-diaminodiphenylmethane, 11.1 g (120 mmol) of epichlorohydrine and 0.36 g of water were mixed and stirred at 80°C for 90 mins. After cooling the admixture to 50°C, 0.23 g of benzyltriethylammonium chloride and 12 ml of a 5N aqueous solution of sodium hydroxide (60 mmol of sodium hydroxide) were added to the admixture and the admixture was stirred for 1 hr at 50°C. The resulting reaction mixture was allowed to stand, whereby the mixture separated into an aqueous layer and an organic layer and the aqueous layer was discarded. The organic layer was washed with water and unreacted epichlorohydrine was distilled out to give 4.13 g of the polyglycidylamine compound in the form of a pale brown liquid.

An infrared absorption spectrum and a $^1$H-NMR spectrum of the thus obtained product are shown in Fig. 3 and Fig. 4, respectively. An epoxy equivalent of the product measured by HCl-pyridine method was 121.

In addition to the ingredients used in the Example, other ingredients can be used in the Example as set forth in the specification to obtain substantially the same results.

## Claims

1. A process for preparing a polyglycidylamine derivative from an aniline compound, characterized by reacting an aniline compound of the formula (I):

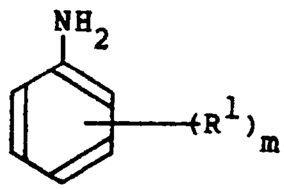

(I)

wherein $R^1$ is methyl and m is 0 or 1, provided that $R^1$ does not bond to p-position, with not less than a stoichometrical amount of an epihalohydrine at a temperature of 50° to 110° C in the presence of water in an amount of 0,1 to 10 moles per 1 mole of the aniline compound to give an addition product, subjecting the addition product to condensation reaction with an aldehyde compound or compounds of the formula (II):

$R^2$-CHO (II)

wherein $R^2$ is a hydrogen atom or an aliphatic group of $C_1$ to $C_{20}$, in the presence of HCl to give a condensation product, and treating the condensation product with alkali in the presence of a phase transfer catalyst to cause ring closure and form epoxy group.

2. A process of claim 1,wherein $R^2$ is hydrogen or an aliphatic group of $C_1$ to $C_3$.

3. A process of claim 1, wherein the aldehyde compound is HCHO, $CH_3CHO$, $CH_3CH_2CHO$,

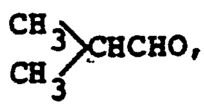

$CH_3CH_2CH_2CH_2CHO$,

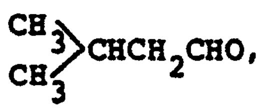

or admixture thereof.

4. A process of any of claims 1 to 3, wherein the aniline compound is aniline, the aldehyde compound is formaldehyde and an obtained polyglycidylamine derivative is N,N,N',N'-tetraglycidyl-4,4'diaminodiphenylmethane.

5. A process of any of claims 1 to 4, wherein the phase transfer catalyst is a quarternary ammonium salt, a quarternary phosphonium salt or a quarternary arsonium salt.

## Revendications

1. Procédé de préparation d'un dérivé de polyglycidylamine à partir d'un composé d'aniline, caractérisé en ce qu'on fait réagir un composé d'aniline de formule (I):

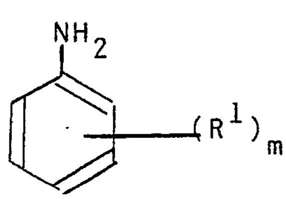

(I)

dans laquelle $R^1$ est un radical méthyle et m est 0 ou 1, à la condition que $R^1$ ne se lie pas à la

position p, avec une quantité qui n'est pas inférieure à la quantité stoechiométrique d'une épihalohydrine à une température de 50 à 110 °C en présence d'eau à raison de 0,1 à 10 moles par mole de composé d'aniline pour obtenir un produit d'addition, on soumet le produit d'addition à une réaction de condensation avec un ou plusieurs composé(s) d'aldéhyde de formule (II) :

$R^2$-CHO (II)

dans laquelle $R^2$ est un atome d'hydrogène ou un groupe aliphatique en $C_{1-20}$, en présence de HCl pour donner un produit de condensation et on traite le produit de condensation avec un alcali en présence d'un catalyseur de transfert de phase pour provoquer la fermeture du noyau et former le groupe époxy.

2. Procédé selon la revendication 1, dans lequel $R^2$ est un atome d'hydrogène ou un radical aliphatique en $C_{1-3}$.

3. Procédé selon la revendication 1, dans lequel le composé d'aldéhyde est HCHO, $CH_3CHO$, $CH_3CH_2CHO$,

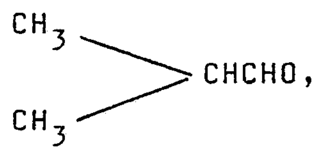

$CH_3CH_2CH_2CH_2CHO$,

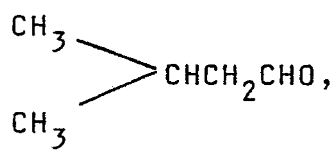

ou leurs mélanges.

4. Procédé selon l'une quelconque des revendications 1 à 3, dans lequel le composé d'aniline est l'aniline ; le composé d'aldéhyde est le formaldéhyde ; et le dérivé obtenu de polyglycidylamine est le N,N,N',N'-tetraglycidyl-4,4'-diaminodiphénylméthane.

5. Procédé selon l'une quelconque des revendications 1 à 4, dans lequel le catalyseur de transfert de phase est un sel d'ammonium quaternaire, un sel de phosphonium quaternaire ou un sel d'arsonium quaternaire.

## Patentansprüche

1. Verfahren zur Herstellung eines Polyglycidylaminderivats aus einer Anilinverbindung, gekennzeichnet durch Reaktion einer Anilinverbindung der Formel (1):

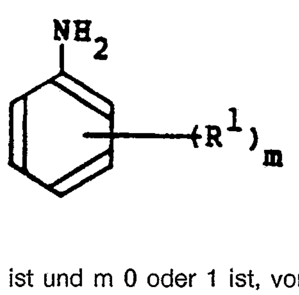

(I)

worin $R^1$ Methyl ist und m 0 oder 1 ist, vorausgesetzt, daß $R^1$ nicht in der p-Position gebunden ist, mit

nicht weniger als einer stöchiometrischen Menge eines Epihalohydrins bei einer Temperatur von 50 bis 110° C in Gegenwart einer Menge von 0,1 bis 10 Mol Wasser pro 1 Mol der Anilinverbindung, unter Bildung eines Additionsproduktes, Unterwerfung des Additionsprodukts der Kondensationsreaktion mit einer Aldehydverbindung oder Verbindungen der Formel (II):

$R^2$-CHO (II)

worin $R^2$ ein Wasserstoffatom oder eine aliphatische Gruppe von $C_1$ bis $C_{20}$ ist, in Gegenwart von HCl unter Bildung eines Kondensationsproduktes, und Behandlung des Kondensationsproduktes mit Alkali in Gegenwart eines Phasentransferkatalysators, um Ringschluß und Bildung einer Epoxygruppe zu bewirken.

**2.** Verfahren nach Anspruch 1, worin $R^2$ Wasserstoff oder eine aliphatische Gruppe von $C_1$ bis $C_3$ ist.

**3.** Verfahren nach Anspruch 1, worin die Aldehydverbindung HCHO, $CH_3CHO$, $CH_3CH_2CHO$,

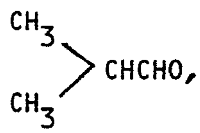

$CH_3CH_2CH_2CH_2CHO$,

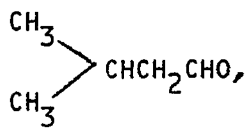

oder Mischung davon ist.

**4.** Verfahren nach jedem der Ansprüche 1 bis 3, worin die Anilinverbindung Anilin ist, die Aldehydverbindung Formaldehyd ist und ein erhaltenes Polyglycidylaminderivat N,N,N',N'-Tetraglycidyl-4,4'-diaminodiphenylmethan ist.

**5.** Verfahren nach jedem der Anspruch 1 bis 4, worin der Phasentransferkatalysator ein quarternäres Ammoniumsalz, ein quarternäres Phosphoniumsalz oder ein quarternäres Arsoniumsalz ist.

FIG. 1

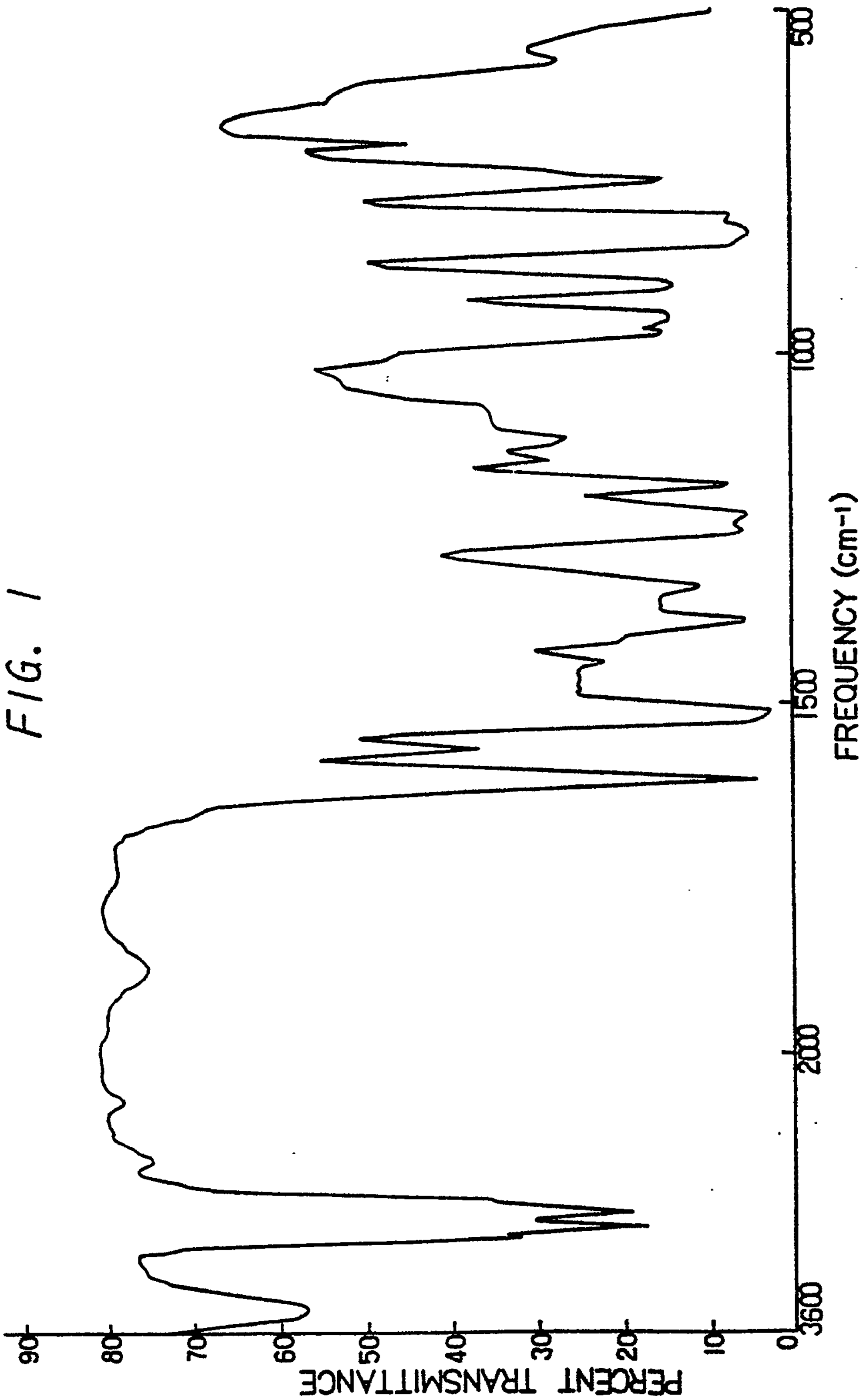
PERCENT TRANSMITTANCE
90
80
70
60
50
40
30
20
10
0
3600
2000
1500
1000
500
FREQUENCY (cm⁻¹)

FIG. 2

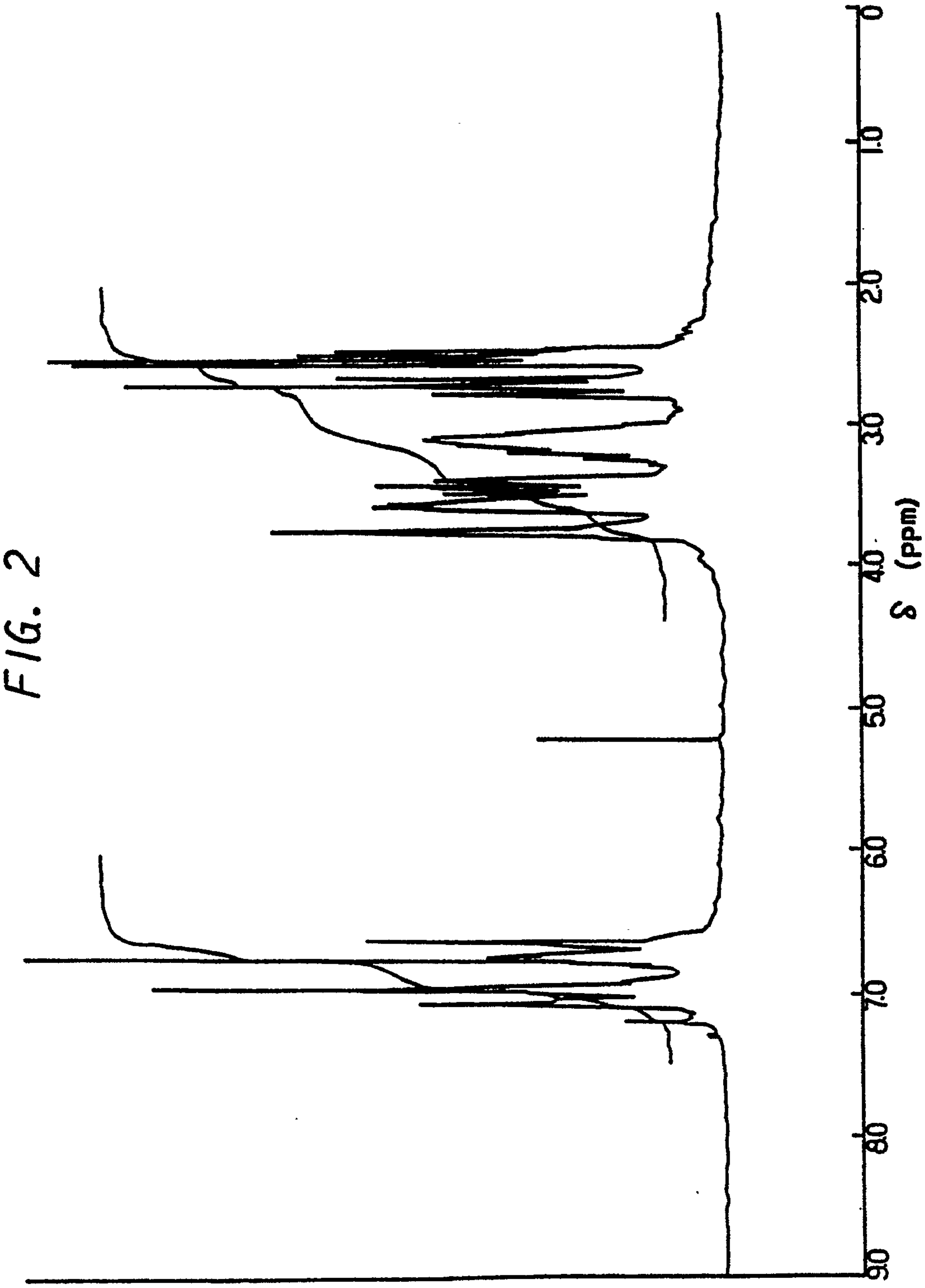

FIG. 3

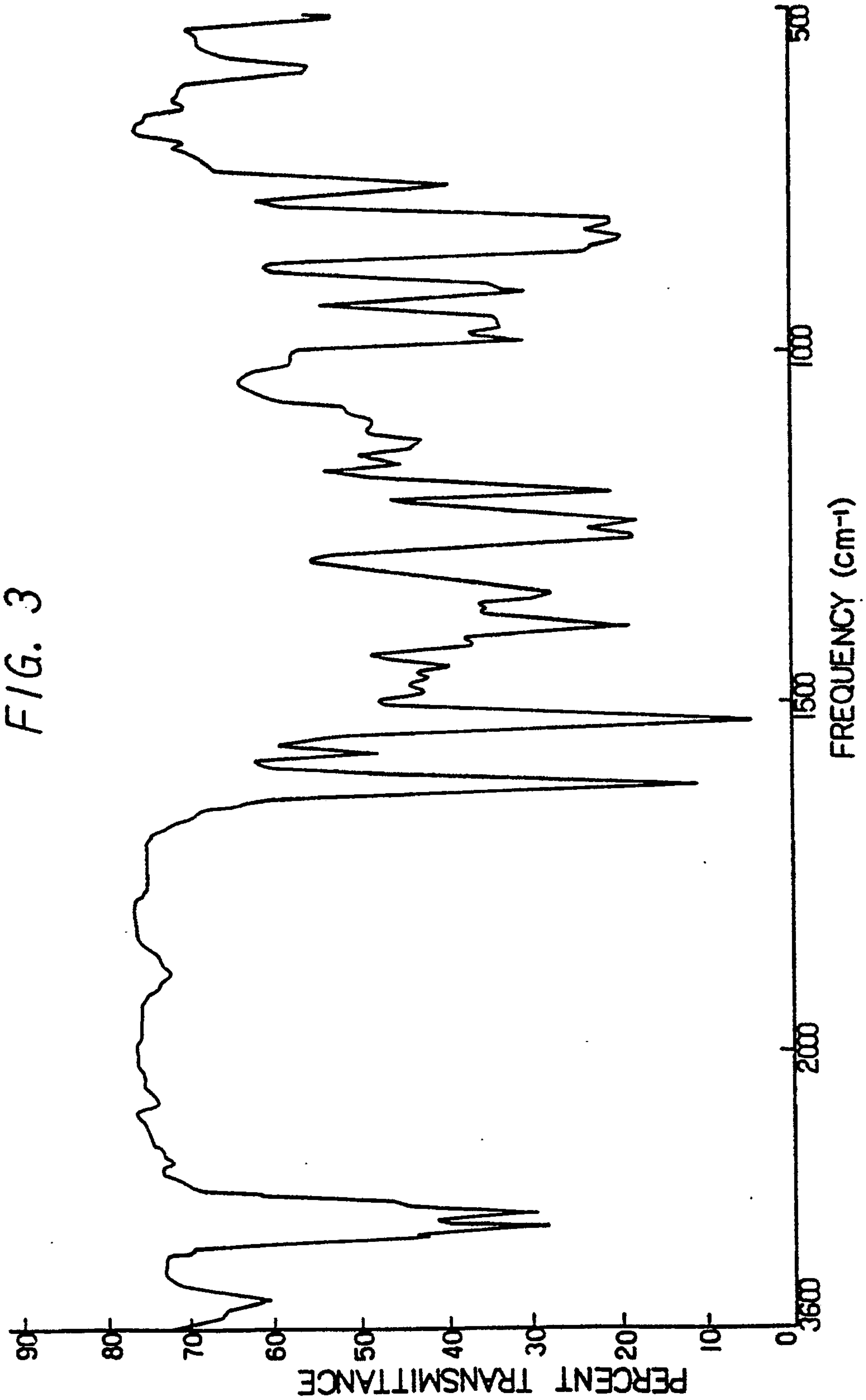
PERCENT TRANSMITTANCE
0
10
20
30
40
50
60
70
80
90
FREQUENCY (cm-1)
3600
2000
1500
1000
500

FIG. 4